# EUROPEAN PATENT SPECIFICATION

(11) **EP 3 417 867 B1**
(45) Date of publication and mention of the grant of the patent: **24.11.2021**
(21) Application number: 17753416.1
(22) Date of filing: 10.02.2017
(51) Int. Cl.: A61K 36/185, A23L 33/105, A61K 9/00

(54) **COMPOSITION CONTAINING MEDICINAL HERB EXTRACT AS ACTIVE INGREDIENT FOR PREVENTING, ALLEVIATING, OR TREATING STRESS OR DEPRESSION**
ZUSAMMENSETZUNG MIT HEILKRÄUTEREXTRAKT ALS WIRKSTOFF ZUR VORBEUGUNG, LINDERUNG ODER BEHANDLUNG VON STRESS ODER DEPRESSIONEN
COMPOSITION CONTENANT UN EXTRAIT DE PLANTE MÉDICINALE COMME PRINCIPE ACTIF POUR LA PRÉVENTION, L'ATTÉNUATION OU LE TRAITEMENT DU STRESS OU DE LA DÉPRESSION

(30) Priority: 16.02.2016 KR 20160017790
(43) Date of publication of application: 26.12.2018
(73) Proprietor: Korea Institute of Oriental Medicine, Daejeon 34054 (KR)
(72) Inventor: LEE, Mi Young, Seoul 06183 (KR); CHOI, Goya, Daejeon 35252 (KR); IM, A Rang, Seoul 03035 (KR); KIM, Young Hwa, Anyang-si Gyeonggi-do 13908 (KR); CHAE, Sung Wook, Daejeon 35230 (KR)
(74) Representative: Hiebl, Inge Elisabeth
(86) International application number: PCT/KR2017/001435
(87) International publication number: WO 2017/142255

(56) References cited:
- WO-A1-2011/069214
- WO-A2-2012/030841
- CN-A- 102 085 167
- FR-A1- 3 004 651
- KR-B1- 101 564 430
- US-A1- 2013 184 359
- US-B2- 8 454 943
- CLÁUDIA VANZELLA ET AL: "Antidepressant-like effects of methanol extract of Hibiscus tiliaceus flowers in mice", BMC COMPLEMENTARY AND ALTERNATIVE MEDICINE, BIOMED CENTRAL LTD., LONDON, GB, vol. 12, no. 1, 12 April 2012 (2012-04-12) , page 41, XP021126598, ISSN: 1472-6882, DOI: 10.1186/1472-6882-12-41
- PALLAVI B. SHEWALE, RUPALI A. PATIL, AND YOGESH A. HIRAY: "Antidepressant-like activity of anthocyanidins from Hibiscus rosa-sinensis flowers in tail suspension test and forced swim test", INDIAN JOURNAL OF PHARMACOLOGY, [Online] vol. 44, no. 4, July 2012 (2012-07), pages 454-457, XP002794156, DOI: 10.4103/0253-7613.99303 Internet Retrieved from the Internet: URL:https://www.ncbi.nlm.nih.gov/pmc/artic les/PMC3469946/> [retrieved on 2019-09-11]
- VANZELLA, C. ET AL.: 'Antidepressant-like Effects of Methanol Extract of Hibiscus Tiliaceus Flowers in Mice' BMC COMPLEMENTARY & ALTERNATIVE MEDICINE vol. 12, no. 41, 2012, pages 1 - 6, XP021126598

## Description

### TECHNICAL FIELD

The present invention relates to a composition for use in a method of preventing, ameliorating, or treating stress or depression comprising an ethanol extract of *Hibiscus syriacus* as an effective ingredient.

### BACKGROUND ART

In accordance with a rapid change and diversification of the society, modern-day people are asked to cover multiple roles. Accordingly, there are more and more people suffering from mental illness like depression, anxiety disorder, or sleep disorder that are caused by various stresses. According to the year 2006 epidemiologic study carried out by Ministry or Health and Welfare of South Korea regarding mental illness in which adults with age of from 18 to 64 are examined as a subject, "one-year prevalence of mental illness" is found to be 17.1% in South Korea. Furthermore, "lifetime prevalence of mental illness", which indicates the proportion of a population who, at some point during their lifetimes, has ever experienced at least one mental illness is 30%, i.e., one in three adults (based on the data of year 2006). In addition, due to the pressure relating to hard study or the like, youth experiencing mental illness tends to increase in recent years.

Stress indicates a state of having physical or psychological tension, which is caused by many factors commonly found in everyday life and it contributes to an occurrence of almost every mental disorder and medical disorder. If not suitably dealt with and left as it is, stress may yield a psychological response like depression, anxiety, fatigue, anger, temperament variation, or the like and also a physiological response such as reduced α-wave among brain waves, increased body pressure and pulse, or the like. As those responses are shown repeatedly, an illness like depression, anxiety, and sleep disorder is caused, a personal loss, a familial loss, and a social loss are yielded, and they become a reason of having lower quality of life.

Among the disorders that are caused by stress, depression indicates a psychological state of a stagnant mood which is characterized by emotional feelings like sadness, despair, and frustration. Namely, it indicates a state developed from "gloomy feeling" as normal feeling to major depressive disorder via a dysthymic disorder. Main symptoms of stress are characterized by a major depressive episode, which is a period exhibiting a stagnant common feeling or a lack of interest or joyfulness in most activities that are combined with a change in appetite, weight, or sleep pattern, psychomotor agitation or retardation, decreased thinking ability, concentration, or ability of making a decision, lack of liveliness and fatigue feeling, feeling of nothingness, self-blaming or guilty feeling, frequent thought of death or suicide, or a plan or an attempt for committing suicide.

*Hibiscus syriacus* is the other name of rose of Sharon (*Hibiscus syriacus* L.), and as a herbal medicine name, *Hibiscus syriacus* skin, *Hibiscus syriacus* flower, *Hibiscus syriacus* root, and *Hibiscus syriacus* seed are used. Rose of Sharon is a deciduous tree belonging to Malvaceae family, and, in South Korea, it is found in Pyeongnam area and a southern part of Kangwon province. According to the Korean Herbal Pharmacopoeia, skins of stems and roots of rose of Sharon are defined as Hibisci Cortex. According to the descriptions of Donguibogam (i.e., a classic book on Korean traditional medicine), it is described as follows: "it has a mild pharmaceutical property without any toxicity, can stop chronic furuncle and bleeding, is effective for thirstiness after diarrhea, and can promote sleeping. The flower has a cold pharmaceutical property without any toxicity, is useful for treating red dysentery and white dysentery, and taken after roasting or as a tea for treating chronic furuncle and bleeding." According to the description of Bonchogangmok (i.e., another classic book on Korean traditional medicine), it is useful for treating red leucorrhea or white leucorrhea of women, stopping pains associated with abscess, yielding clear eyes when washed with water boiled with plant as an agent for treating scabies, and also promoting blood circulation.

As an exemplary technique relating to an extract of *Hibiscus syriacus* root or *Hibiscus syriacus* flower that is known to date, it is described in Korean Patent Publication No. 2005-0080428 that *Hibiscus syriacus* is effective for treating migraine. In Korean Patent Registration No. 1451952, a composition and a food for treating hangover containing *Hibiscus syriacus* root is disclosed. Furthermore, in Korean Patent Application Publication No. 2010-0059302, it is described that an extract of *Hibiscus syriacus* skin is effective for skin moisturization, wound healing, and anti-aging. However, so far there is no disclosure of a composition for preventing, ameliorating, or treating a stress disorder, in particular, depression caused by stress, comprising an extract of *Hibiscus syriacus* flower or *Hibiscus syriacus* root as an effective ingredient as it is described in the present invention.

Cláudia Vanzella et al.: "Antidepressant-like effects of methanol extract of Hibiscus tiliaceus flowers in mice", BMC COMPLEMENTARY AND ALTERNATIVE MEDICINE, BIOMED CENTRAL LTD., LONDON, GB, vol. 12, no. 1, 12 April 2012, page 41, discloses an antidepressant-like profile of action for the extract of Hibiscus tiliaceus without sedative side effect since the methanol extract of H. tiliaceus did not potentiate the effect of ketamine-induced hypnosis, moreover, this extract given alone did not produce any sedation per se.

Pallavi B. Shewale et al.: "Antidepressant-like activity of anthocyanidins from Hibiscus rosa-sinensis flowers in tail suspension test and forced swim test", INDIAN JOURNAL OF PHARMACOLOGY, [Online] vol. 44, no. 4, July 2012, pages 454-457, discloses the antidepressant activity of a methanol extract containing anthocyanins and anthocyanidins of H. rosa-sinensis flowers in mice.

### DETAILED DESCRIPTION OF THE INVENTION

### TECHNICAL PROBLEMS TO BE SOLVED

The present invention is devised under the circumstances described above. The present invention relates to a composition for preventing or treating stress or depression comprising medicinal herb extract as an effective ingredient. Specifically, by confirming that an extract of *Hibiscus syriacus* flower and an extract of *Hibiscus syriacus* root as an effective ingredient of the present invention have an activity of protecting nerve cells, can reduce the expression amount of an inflammatory factor, and exhibit an anti-depression effect in an animal model induced to have depression caused by stress, the present invention is completed.

### TECHNICAL MEANS FOR SOLVING THE PROBLEMS

To achieve the purpose described above, the present invention provides a pharmaceutical composition for use in a method of preventing or treating stress disorder comprising an ethanol extract of *Hibiscus syriacus* as an effective ingredient.

The present invention further provides a functional health food composition for use in a method of preventing or ameliorating stress or depression caused by stress comprising an ethanol extract of *Hibiscus syriacus* as an effective ingredient.

### ADVANTAGEOUS EFFECT OF THE INVENTION

The present invention relates to a pharmaceutical composition for use in a method of preventing, ameliorating, or treating stress or depression caused by stress comprising an ethanol extract of *Hibiscus syriacus* as an effective ingredient. Specifically, it is shown that an extract of *Hibiscus syriacus* flower or *Hibiscus syriacus* root has an activity of protecting nerve cells, can reduce the expression amount of an inflammatory factor that is increased by stress, and is effective for depression caused by stress.

### BRIEF DESCRIPTION OF THE DRAWINGS

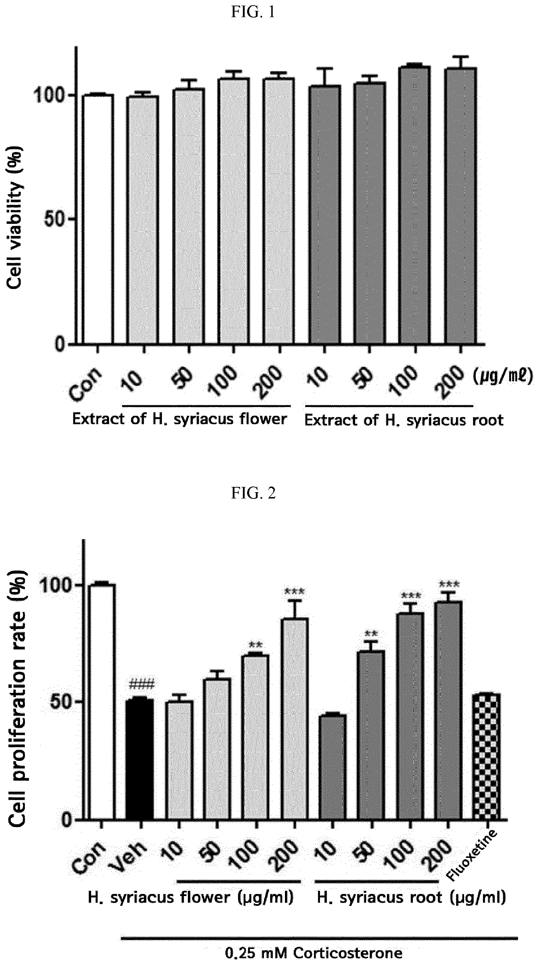

Fig. 1 is a result of determining the cell viability (%) in the presence of an extract of *Hibiscus syriacus* flower or *Hibiscus syriacus* root.
Fig. 2 is a result of determining the activity of protecting nerve cells by an extract of *Hibiscus syriacus* flower or *Hibiscus syriacus* root after a treatment with corticosterone. Con is a normal group representing cells which have not been treated with any agent, and Veh is a control group treated with corticosterone. ### indicates that the cell proliferation is reduced in the corticosterone treatment group in a statistically significant sense compared to the normal group (Con), in which p<0.001. ** and *** indicate that the difference in cell proliferation has a statistically significant sense between the group treated with an extract of *Hibiscus syriacus* flower or *Hibiscus syriacus* root and the control group (Veh), in which **means p<0.01 and ***means p<0.001. Fluoxetine is a positive control group, i.e., treatment with 10 µM fluoxetine.

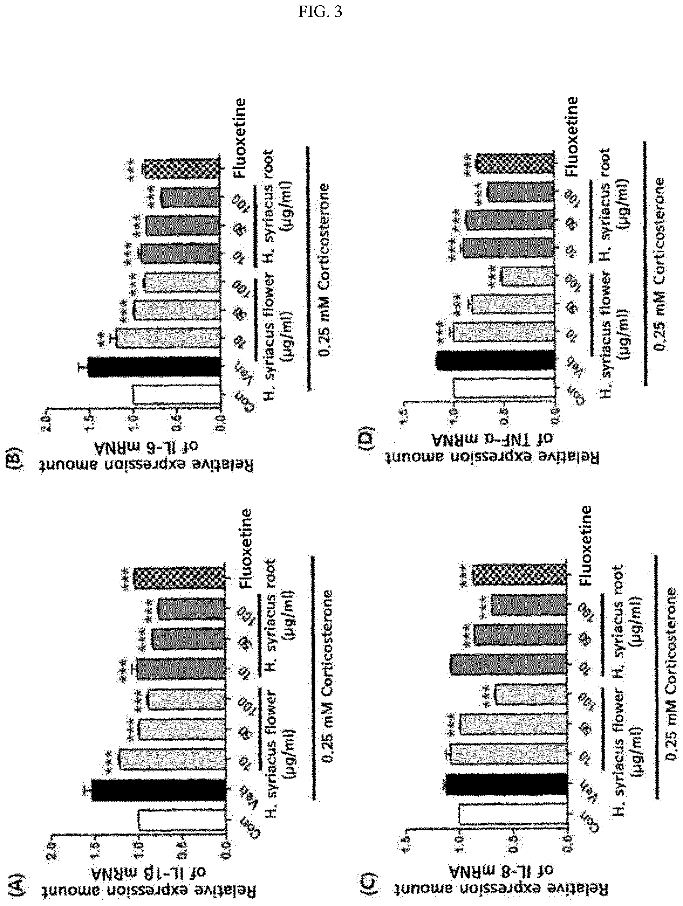

Fig. 3 is a result of determining a change in the mRNA expression amount of an intracellular inflammatory factor caused by an extract of *Hibiscus syriacus* flower or *Hibiscus syriacus* root after a treatment with corticosterone. Fig. 3(A) shows a change in the mRNA expression amount of IL-1β, Fig. 3(B) shows a change in the mRNA expression amount of IL-6, Fig. 3(C) shows a change in the mRNA expression amount of IL-8, and Fig. 3(D) shows a change in the mRNA expression amount of TNF-α. ** and ***indicate that, compared to the Veh group treated with corticosterone alone, the mRNA expression amount is reduced in a statistically significant sense in the group which has been pretreated with an extract of *Hibiscus syriacus* flower or *Hibiscus syriacus* root before the addition of corticosterone, in which ** means p<0.01 and *** means p<0.001.

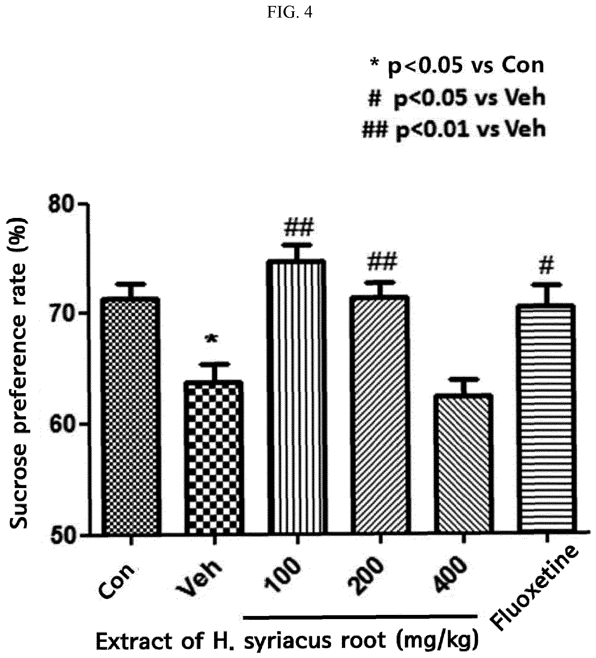

Fig. 4 is a result of determining the influence of an extract of *Hibiscus syriacus* root on improvement of depression behavior, in which the determination is made by using sucrose preference test (SPT) after an exposure to immobility stress. * indicates that the control group with stress (Veh) has a sucrose intake amount that is reduced in statistically significant sense compared to the normal group (Con), in which p<0.05. # and ## indicate that, compared to the control group applied with the stress only (Veh), the sucrose intake amount has increased in statistically significant sense in the group which has been orally administered with an extract of *Hibiscus syriacus* root in which # means p<0.05 and ## means p<0.01.

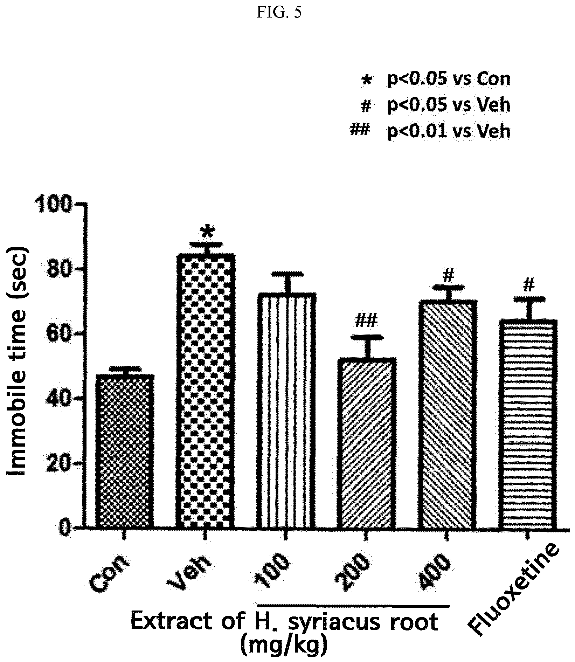

Fig. 5 is a result of determining the influence of an extract of *Hibiscus syriacus* root on improvement of depression behavior, in which the determination is made by using forced swimming test (FST) after an exposure to immobility stress. *indicates that the control group with stress only (Veh) has continuous immobility state time that has increased in statistically significant sense compared to the normal group (Con), in which p<0.05. # and ## indicate that, compared to the control group applied with the stress only (Veh), the continuous immobility state time has decreased in statistically significant sense in the group which has been orally administered with an extract of *Hibiscus syriacus* root in which # means p<0.05 and ## means p<0.01.

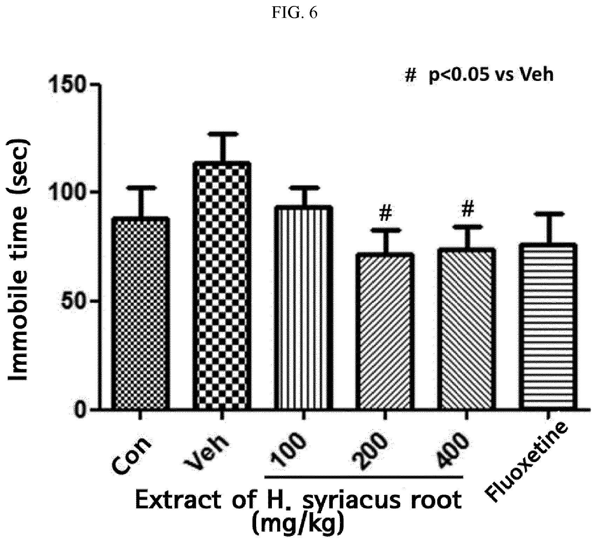

Fig. 6 is a result of determining the influence of an extract of *Hibiscus syriacus* root on improvement of depression behavior, in which the determination is made by using tail suspension test (TST) after an exposure to immobility stress. # indicates that, compared to the control group applied with the stress only (Veh), the continuous immobility state time has decreased in statistically significant sense in the group which has been orally administered with an extract of *Hibiscus syriacus* root in which # means p<0.05.

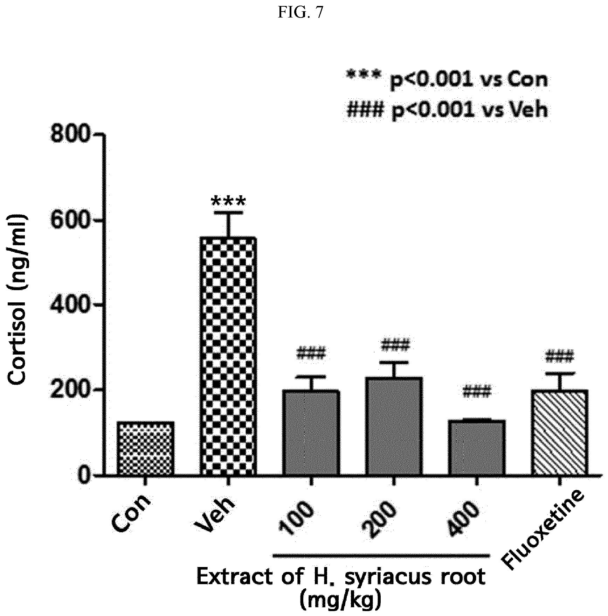

Fig. 7 is a result of determining the influence of an extract of *Hibiscus syriacus* root on stress hormone, in which the determination is made by measuring the blood cortisol concentration of a mouse after an exposure to immobility stress. ***indicates that the control group applied with the stress (Veh) has cortisol production that has increased in statistically significant sense compared to the normal group, in which p<0.001. ### indicates that, compared to the control group applied with the stress only (Veh), the cortisol production has decreased in statistically significant sense in the group which has been orally administered with an extract of *Hibiscus syriacus* root in which ### means p<0.001.

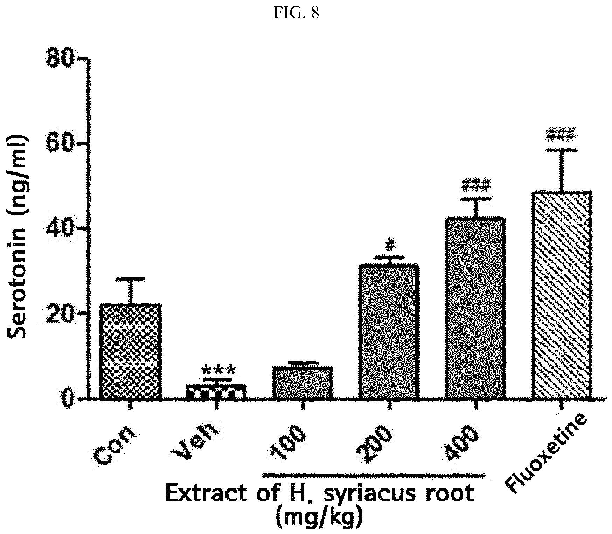

Fig. 8 is a result of determining the influence of an extract of *Hibiscus syriacus* root on a neuronal transmitter, in which the determination is made by measuring the blood serotonin concentration of a mouse after an exposure to immobility stress. # and ### indicate that, compared to the control group applied with the stress only (Veh), serotonin has increased in statistically significant sense in the group which has been orally administered with an extract of *Hibiscus syriacus* root in which # means p<0.05 and ### means p<0.001.

### BEST MODE(S) FOR CARRYING OUT THE INVENTION

The present invention relates to a pharmaceutical composition for use in a method of preventing or treating stress disorder comprising an ethanol extract of *Hibiscus syriacus* as an effective ingredient. The extract of *Hibiscus syriacus* is preferably extracted from *Hibiscus syriacus* flower, *Hibiscus syriacus* root, or a whole plant of *Hibiscus syriacus,* and more preferably extracted from *Hibiscus syriacus* flower or *Hibiscus syriacus* root, but it is not limited thereto.

The stress order is preferably at least one selected from depression, sleep disorder, or anxiety disorder that are caused by stress, and more preferably depression caused by stress, but it is not limited thereto.

With regard to a method for preparing an ethanol extract of *Hibiscus syriacus* of the present invention, any kind of common methods that are generally known as an extraction method in the pertinent art, e.g., filtration, hot water extraction, impregnation extraction, extraction by reflux condensation, and ultrasonic extraction, can be used. It is preferable that the extraction is carried out by adding the extraction solvent in an amount of 2 to 40 times the volume of dried *Hibiscus syriacus.* The extraction temperature is preferably between 20°C and 100°C, and most preferably 80°C and 95°C, but it is not limited thereto. Furthermore, the extraction time is preferably between 0.5 hour and 10 hours, more preferably between 1 hour and 4 hours, and most preferably 3 hours, but it is not limited thereto. Concentration is preferably concentration under reduced pressure which uses a vacuum condenser or a vacuum rotary evaporator, but it is not limited thereto. Furthermore, the drying is preferably carried out by drying under reduced pressure, drying under vacuum, drying under boiling, spray drying, or freeze drying, but it is not limited thereto.

The pharmaceutical composition for the use of the present invention may be prepared in various formulations including an oral formulation and a parenteral formulation. In case of producing a preparation, production is made by using a diluent or a vehicle such as filler, bulking agent, binding agent, moisturizing agent, disintegrating agent, or surfactant that are commonly used for producing a preparation, but it is not limited thereto.

As for the solid preparation for oral administration, a tablet, a pill, a powder preparation, a granule, a capsule or the like are included, and such solid preparation is produced by mixing at least one compound with one or more vehicles such as starch, calcium carbonate, sucrose, lactose, or gelatin. Furthermore, other than simple vehicles, a lubricating agent such as magnesium stearate or talc is also used. For the liquid preparation for oral administration, a suspension, a solution preparation for internal use, an emulsion, a syrup preparation, or the like can be mentioned. Other than water or liquid paraffin as a commonly used simple diluent, various kinds of a vehicle such as moisturizing agent, sweetening agent, aromatic agent, or preservatives may be included.

Examples of a preparation for parenteral administration include a sterilized aqueous solution, a non-soluble agent, a suspension agent, an emulsion, a freeze-drying agent, and a suppository agent. As a water insoluble solvent or a suspending agent, propylene glycol, polyethylene glycol, or vegetable oil such as olive oil, and injectable ester such as ethylolate can be used. As a base for a suppository, witepsol, macrogol, tween 61, cacao fat, laurin fat, glycerol, gelatin, or the like can be used.

The pharmaceutical composition for the ues of the present invention can be administered either orally or parenterally. In case of parenteral administration, it is preferable to choose external application on skin, intraperitoneal, rectal, intravenous, muscular, subcutaneous, endometrium injection, or intracerebroventricular injection, but it is not limited thereto.

The pharmaceutical composition for the use of the present invention is administered in a pharmaceutically effective amount. As described herein, the expression "pharmaceutically effective amount" means an amount sufficient for treating a disorder at reasonable benefit-risk ratio that can be applied for a medical treatment. The effective dose level may be determined based on a type or severeness of a disorder, activity of a pharmaceutical, sensitivity to a pharmaceutical, administration period, administration route, excretion ratio, time period for therapy, elements including a pharmaceutical used in combination, and other elements that are well known in the medical field. The composition for the use of the present invention can be administered as a separate therapeutic agent, or it can be used in combination with other therapeutic agent. It can be administered in order or simultaneously with a conventional therapeutic agent. It can be also administered as single-dose or multidose. It is important to administer an amount which allows obtainment of the maximum effect with minimum dose while considering all of the aforementioned elements without having any side effect, and the dosage can be easily determined by a person skilled in the pertinent art.

The dosage of the composition for the use of the present invention may vary depending on bodyweight, age, sex, health state, diet of a patient, administration period, administration method, excretion rate, and severeness of disorder. However, the daily dosage is, in terms of the amount of an extract of *Hibiscus syriacus* root, 0.01 to 1,000 mg/kg, preferably 30 to 500 mg/kg, and more preferably 50 to 300 mg/kg, and it can be administered 1 to 6 times per day. However, since the dosage may be increased or decreased depending on the administration route, severeness of obesity, sex, body weight, age or the like, the scope of the present invention is not limited by the aforementioned dosage in any sense.

The present invention further relates to a functional health food composition for use in a method of preventing or ameliorating stress or depression caused by stress comprising an ethanol extract of *Hibiscus syriacus* as an effective ingredient. The extract of *Hibiscus syriacus* is preferably extracted from *Hibiscus syriacus* flower, *Hibiscus syriacus* root, or a whole plant of *Hibiscus syriacus,* and more preferably extracted from *Hibiscus syriacus* flower or *Hibiscus syriacus* root, but it is not limited thereto.

The functional health food composition for the use of the present invention comprising an ethanol extract of *Hibiscus syriacus* as an effective ingredient may be directly added to a food product or used with other food product or food ingredient, and it can be suitably used according to a common method. The mixing amount of the effective ingredient can be suitably determined based on the purpose of use (i.e., prevention or amelioration). In general, the amount of an extract of *Hibiscus syriacus* to be comprised in the functional health food composition can be 0.1 to 90 parts by weight relative to the total weight of the functional health food composition. However, in case of long-term consumption under the purpose of maintaining good health and hygiene or managing health, it can be an amount below the aforementioned range, and, as there is no problem in terms of safety, the effective ingredient may be also used in an amount above the aforementioned range.

When the functional health food composition for the use of the present invention is consumed in the form of a beverage, other ingredients are not particularly limited except that, as an essential ingredient, the aforementioned extract of *Hibiscus syriacus* is comprised at indicated ratio, and, like common beverages, various flavors or natural carbohydrates can be comprised as an additional component. Examples of the natural carbohydrates include monosaccharides such as glucose or fructose, disaccharides such as maltose or sucrose, polysaccharides such as dextrin or cyclodextrin, and sugar alcohols such as xylitol, sorbitol, or erythritol. As a flavor other than those described above, natural flavor (taumatin, stevia extract (e.g., rebaudioside A and glycyrrhizin)) and synthetic flavor (e.g., saccharine and aspartame) can be advantageously used.

The functional health food composition for the use of the present invention may further comprise, other than the effective ingredient, at least one selected from a nutritional supplement, a vitamin, an electrolyte, a flavor, a coloring agent, an enhancing agent, pectinic acid and a salt thereof, alginic acid and a salt thereof, an organic acid, protective colloidal thickening agent, a pH adjusting agent, a stabilizer, a preservative, glycerin, alcohol, and a carbonating agent used for carbonated drink. Other than those, fruit flesh for producing natural fruit juice or vegetable drink can be comprised in the functional health food composition of the present invention. The fruit flesh may be used either independently or in combination thereof. Ratio of the above various additives is not critical, but it is generally selected from a range of about 0.1 to 20 parts by weight relative to 100 parts by weight of the extract of *Hibiscus syriacus* of the present invention.

Hereinbelow, the present invention is explained in greater detail in view of the Examples.

### Example 1. Preparation of extract of Hibiscus syriacus flower or Hibiscus syriacus root

The *Hibiscus syriacus* flower used in the present invention was collected from the area of Daejon in Korea, and roots of rose of Sharon were purchased from a tree farm in Youngchon of Kyungbuk in Korea and used as *Hibiscus syriacus* root. The *Hibiscus syriacus* flower (250 g) was added with 30% (v/v) ethanol (20x volume) and extracted under reflux at 90±2°C for 3 hours. Furthermore, 1.8 kg of the *Hibiscus syriacus* root were added with 70% (v/v) ethanol (10x volume) and extracted under reflux at 84±2°C for 3 hours. After the first filtering using a filtering wire, the second filtering was carried out by using cotton to have a concentrate, which was then subjected to freeze-drying followed by pulverization. After the concentration, the *Hibiscus syriacus* flower was obtained at a yield of 27.6%, and the *Hibiscus syriacus* root was obtained at a yield of 7.2%.

### Example 2. Cell culture

SK-N-SH cells as human neuroblastoma cell line were obtained from Korean Cell Line Bank, cultured in DMEM (Dulbecco's modified Eagle's medium) medium containing 10% bovine serum and 1% penicillin/streptomycin (37°C, 5% CO₂ incubator), and used. Once a cell monolayer is formed, the cells were treated with TrypLE 1 × solution to release the cells followed by subculture.

### Example 3. Determination of cytotoxicity and measurement of activity of protecting nerve cells

Cytotoxicity of an extract of *Hibiscus syriacus* flower and *Hibiscus syriacus* root, which is an effective ingredient of the present invention, was determined in human neuroblastoma cell. On a Corning BioCoat collagen I 96-well plate, SK-N-SH cells were sewn at 3 × 10⁴/well. Thereafter, the cells were treated for 24 hours with an extract of *Hibiscus syriacus* flower or *Hibiscus syriacus* root at a concentration of 10, 50, 100, or 200 µg/ml. Then, MTS assay was carried out and, by measuring the absorbance using ELISA (enzyme-linked immunosorbent assay) plate reader, the cell viability was determined.

As a result, it was confirmed that the extract of *Hibiscus syriacus* flower or *Hibiscus syriacus* root exhibits absolutely no cytotoxicity as it is shown in Fig. 1.

Furthermore, based on the toxicity of corticosterone, the activity of protecting nerve cells in human blastoma was examined. Specifically, on a Corning BioCoat collagen I 96-well plate, SK-N-SH cells were sewn at 3×10⁴/well. Thereafter, the cells were pre-treated for 24 hours with an extract of *Hibiscus syriacus* flower or *Hibiscus syriacus* root at a concentration of 10, 50, 100, or 200 µg/ml followed by a treatment with 0.25 mM corticosterone for 1 hour to induce oxidative stress. Then, MTS assay was carried out and, by measuring the absorbance using ELISA (enzyme-linked immunosorbent assay) plate reader, the cell proliferation was determined.

As a positive control, a treatment with 10 µM fluoxetine (Sigma, St. Louis, MO, USA) was carried out instead of the treatment with an extract of *Hibiscus syriacus* flower or *Hibiscus syriacus* root.

As a result, it was confirmed that an extract of *Hibiscus syriacus* flower and an extract of *Hibiscus syriacus* root, which are an effective ingredient of the present invention, have an activity of protecting nerve cells against the toxicity of corticosterone. As it is shown in Fig. 2, it was confirmed that the cells were diminished by 50% or so according to a treatment with corticosterone, and when the treatment is carried out by simultaneously using the effective ingredient of the present invention with corticosterone, dose-dependent cell proliferation (%) is shown from the treatment using 50-200 µg/ml extract of *Hibiscus syriacus* flower or *Hibiscus syriacus* root when compared to the treatment with corticosterone only. In addition, more effective cell proliferation than fluoxetine, i.e., positive control, was shown.

### Example 4. Determination of effect of suppressing mRNA expression of cellular inflammatory factor

In order to confirm the suppressed expression of a cellular inflammatory factor, in which the expression has been increased by corticosterone, SK-N-SH cells were sewn in a 6-well plate, pre-treated for 24 hours with an extract of *Hibiscus syriacus* flower or *Hibiscus syriacus* root at a concentration of 10, 50, or 100 µg/ml followed by a treatment with 0.25 mM corticosterone for 1 hour to induce oxidative stress. Then, RNA was extracted from the cells, and by using a reverse-transcription kit, complementary DNA was obtained. By using TaqMan RNA assay (Applied Biosystems), amplification was carried out with real-time gene expression system (QuantStudio 6, Applied Biosystems), and then RNA was quantified. For the mRNA gene expression, TaqMan probe (FAM dye-labeled, ABI, USA) was used while human actin probe was used as an internal standard. As a positive control, a treatment with 10 µM fluoxetine (Sigma, St. Louis, MO, USA) was carried out instead of the treatment with an extract of *Hibiscus syriacus* flower or *Hibiscus syriacus* root.

As a result, the extract of *Hibiscus syriacus* flower or *Hibiscus syriacus* root exhibited the effect of suppressing the inflammatory factor, which has been increased by corticosterone, as it is shown in Fig. 3. It was found that, according to a treatment with corticosterone, the gene expression amount of IL-1β, IL-6, IL-8, and TNF-α, which are an inflammation-related factor, has increased compared to the normal group, and the group treated with an extract of *Hibiscus syriacus* flower or *Hibiscus syriacus* root showed a reduced gene expression amount.

### Example 5. Determination of anti-depression effect of extract of Hibiscus syriacus root using animal model with immobility stress

### 1) Test animal

As a test animal, 7-week old male C57BL/6 mouse was used after obtaining it from Samtako Bio Korea. The test animals were acclimated for 7 days in the test animal research center, which is a specific pathogen-free facility of Korea Institute of Oriental Medicine. They were divided into the followings 6 groups in total: normal group not exposed to any stress (Control, n=16), stress control group applied with the stress only (Vehicle, n=16), positive control group applied with the stress and commercially available anti-depressant (Fluoxetine, n=16), and test groups applied with the stress and extract of *Hibiscus syriacus* root (100, 200, or 400 mg/kg, n=16). The test methods were reviewed by Test animal ethics committee of Korea Institute of Oriental Medicine (approval number: 15-102), and as for the breeding and maintenance of the test animals, the animals were placed in an IVC (individually ventilated cage) (5 animals per cage), which has been constantly maintained at temperature of 23±1°C, humidity of 50±10% or so, and light/dark period of 12 hours, based on the regulations for management and use of animals by the Test Animal Research Center of Korea Institute of Oriental Medicine.

As a positive control group, fluoxetine was dissolved in physiological saline to 20 mg/kg, and every day for 22 days, it was orally administered to an animal at 11 AM. As a control group applied with the stress only with no administration of any pharmaceuticals, the same amount of physiological saline (300 µl) was orally administered. As for the fluoxetine, the product of Sigma Aldrich (St. Louis, MO, USA) was used.

### 2) Immobility Stress

In Example 5, the immobility stress was applied for 22 days to the control group and test group, and, for the test group, administration of an extract of *Hibiscus syriacus* root at various concentrations was also carried out. The immobility stress was applied to all groups except the normal group as follows: mouse was added to a 50 ml conical tube (one animal per tube), in which holes are created to allow animal breathing, so as to retrain the animal's movement, and the stress was applied for 2 hours starting from 1 PM, which is 2 hours after the administration of pharmaceuticals. The normal group (i.e., control) was kept in another cage so as to avoid any contact with the group to which stress is applied, and other than the deprivation of food and water one day before the forced swimming test and tail suspension test, the animals were allowed to have free access to food and water.

### 3) Sucrose preference test (SPT)

The sucrose preference test was carried out on Day 1 of the test and after completion of the test. During the period in which intake of sucrose solution is examined, 8 animals from each test group were kept in a separate cage, and, to allow recognition of 2% sucrose solution, the sucrose solution was added to 2 water bottles so that the animals can freely taste it. After 24 hours, the solution in one water bottle was exchanged with physiological saline, and 24 hours thereafter, positions of the physiological saline bottle and sucrose solution bottle were switched so as to avoid animal's intake preference based on position. The intake preference was calculated as follows.

As a result, the stress control group (Vehicle) showed a sucrose intake amount that is reduced in significant sense than the normal group (Control), as it is shown in Fig. 4. On the contrary, the positive control group (Fluoxetine) showed the intake amount which has increased in significant sense compared to the stress control group (Vehicle). Furthermore, a significant increase in sucrose intake amount was shown also from the group which has been treated with 100 or 200 mg/kg extract of *Hibiscus syriacus* root.

### 4) Forced Swimming Test (FST)

To measure the anti-depression effect of an extract of *Hibiscus syriacus* root, the forced swimming test was carried out, after the completion of the immobility stress test, by using the method of Porsolt, et. al. (1997). Specifically, after filling water (750 ml) at temperature of 23±1°C in a 1 liter beaker, the test animal was added to the water bath (one animal per bath). Of total 6 minutes, the measurement was not carried out for initial 2 minutes as an acclimation period, while the immobile time of the test animal was measured for the last 4 minutes. The immobility is defined as a state in which the animal stands upright with minimal movement for exposing the head above water, and a change in the immobile time was taken as a yardstick of improved depression-related behavior.

As a result, the control group (Vehicle) showed remarkably increased immobile time compared to the normal group (Control), as it is shown in Fig. 5. On the contrary, the positive control group (Fluoxetine) showed the immobile time which has decreased in significant sense compared to the control group (Vehicle). Furthermore, a significant decrease in immobile time was shown also from the group which has been treated with 200 or 400 mg/kg extract of *Hibiscus syriacus* root compared to the control group (Vehicle).

### 5) Tail Suspension Test (TST)

The method by Can and others was used for the tail suspension test. Specifically, after attaching a fixing device 1 cm apart from the end of the tail of a mouse for test, the mouse was suspended 50 cm apart from the ground. Of total 6 minutes, the measurement was not carried out for initial 2 minutes as an acclimation period, while the immobile time of the test animal was measured for the last 4 minutes. The immobility is defined as a state in which the animal in suspending state remains completely still without showing any movement.

As a result, the control group (Vehicle) showed increased immobile time compared to the normal group (Control), as it is shown in Fig. 6. On the contrary, the positive control group (Fluoxetine) showed the immobile time which has decreased compared to the control group (Vehicle). Furthermore, a significant decrease in immobile time was shown also from the group which has been treated with 200 or 400 mg/kg extract of *Hibiscus syriacus* root compared to the control group (Vehicle).

### 6) Cortisol measurement

Upon the completion of the test, the test animal was anesthetized, and blood was collected from the heart and centrifuged (1,000xg, 15 minutes) to obtain blood serum. Cortisol measurement was carried out by competitive enzyme immunoassay according to the manufacturer's protocol (Cayman chemical, MI, USA). On a 96-well plate, 50 µl of reference solution or sample were added, and the reaction was allowed to occur for 1 day at 4°C. After washing with a washing solution, 200 µl of Ellman's Reagent were added thereto, and after covering with a film, the reaction was allowed to occur for 2 hours or so. Then, the absorbance at 405 nm was measured by using Micro Spectrophotometer (Molecular Device, Sunnyvale, CA, USA).

As a result of measuring cortisol production in blood of mouse, the control group (Vehicle) showed remarkably increased cortisol production compared to the normal group (Control), as it is shown in Fig. 7. On the contrary, the positive control group (Fluoxetine) showed the cortisol production which has decreased in significant sense compared to the control group (Vehicle). Furthermore, a significant decrease in cortisol production was shown also from the group which has been treated with 100, 200, or 400 mg/kg extract of *Hibiscus syriacus* root compared to the control group (Vehicle).

### 7) Serotonin measurement

Upon the completion of the test, the test animal was anesthetized, and blood was collected from the heart and centrifuged (1,000xg, 15 minutes) to obtain blood serum. Serotonin measurement was carried out by competitive enzyme immunoassay according to the manufacturer's protocol (Abcam Inc., Cambridge, UK). A sample was prepared according to 20x dilution of the mouse blood serum in assay buffer, and on a 96-well plate, 100 µl of reference solution or sample, 50 µl of serotonin alkaline phosphatase conjugate, and 50 µl of serotonin antibody were added, and the reaction was allowed to occur for 2 hours at room temperature. Then, 200 µl of pNPP substrate were added, and the reaction was allowed to occur for 1 hour at room temperature. After adding the stop solution (50 µl), the absorbance at 405 nm was measured by using Micro Spectrophotometer (Molecular Device, Sunnyvale, CA, USA).

As a result, the control group (Vehicle) showed decreased serotonin production compared to the normal group (Control), as it is shown in Fig. 8. On the contrary, the positive control group (Fluoxetine) showed the serotonin production which has increased in significant sense compared to the control group (Vehicle). Furthermore, a significant increase in serotonin production was shown also from the group which has been treated with 200 or 400 mg/kg extract of *Hibiscus syriacus* root compared to the control group (Vehicle)

## Claims

1. A pharmaceutical composition for use in a method of preventing or treating stress disorder comprising an ethanol extract of *Hibiscus syriacus* as an effective ingredient.

2. The pharmaceutical composition for the use according to Claim 1, wherein the ethanol extract of *Hibiscus syriacus* is an ethanol extract of *Hibiscus syriacus* flower or *Hibiscus syriacus* root.

3. The pharmaceutical composition for the use according to Claim 1 or 2, wherein the stress disorder is depression caused by stress.

4. The pharmaceutical composition for the use according to any of Claims 1 to 3, wherein it further comprises a vehicle or a diluent other than the effective ingredient.

5. The pharmaceutical composition for the use according to any of Claims 1 to 4, wherein the composition is administered either orally or parenterally.

6. A functional health food composition for use in a method of preventing or ameliorating stress or depression caused by stress comprising an ethanol extract of *Hibiscus syriacus* as an effective ingredient.

7. The functional health food composition for the use according to Claim 6, wherein the ethanol extract of *Hibiscus syriacus* is an ethanol extract of *Hibiscus syriacus* flower or *Hibiscus syriacus* root.

8. The functional health food composition for the use according to Claim 6 or 7, wherein it further comprises, other than the effective ingredient, at least one selected from a nutritional supplement, a vitamin, an electrolyte, a flavor, a coloring agent, an enhancing agent, pectinic acid and a salt thereof, alginic acid and a salt thereof, an organic acid, protective colloidal thickening agent, a pH adjusting agent, a stabilizer, a preservative, glycerin, alcohol, and a carbonating agent used for carbonated drink.

## Patentansprüche

1. Pharmazeutische Zusammensetzung zur Verwendung in einem Verfahren zur Verhütung oder Behandlung einer Stressstörung, umfassend einen Ethanolextrakt aus *Hibiscus syriacus* als Wirkstoff.

2. Pharmazeutische Zusammensetzung zur Verwendung nach Anspruch 1, wobei der Ethanolextrakt aus *Hibiscus syriacus* ein Ethanolextrakt aus *Hibiscus-syriacus-*Blüten oder *Hibiscus-syriacus-*Wurzel ist.

3. Pharmazeutische Zusammensetzung zur Verwendung nach Anspruch 1 oder 2, wobei die Stressstörung eine durch Stress verursachte Depression ist.

4. Pharmazeutische Zusammensetzung zur Verwendung nach einem der Ansprüche 1 bis 3, wobei sie weiter einen Träger oder ein Verdünnungsmittel neben dem Wirkstoff enthält.

5. Pharmazeutische Zusammensetzung zur Verwendung nach einem der Ansprüche 1 bis 4, wobei die Zusammensetzung entweder oral oder parenteral verabreicht wird.

6. Funktionelle Gesundheitslebensmittelzusammensetzung zur Verwendung in einem Verfahren zur Verhütung oder Linderung von Stress oder Depression, verursacht durch Stress, umfassend einen Ethanolextrakt aus *Hibiscus syriacus* als Wirkstoff.

7. Funktionelle Gesundheitslebensmittelzusammensetzung zur Verwendung nach Anspruch 6, wobei der Ethanolextrakt aus *Hibiscus syriacus* ein Ethanolextrakt aus *Hibiscus-syriacus-*Blüten oder *Hibiscus-syriacus-*Wurzel ist.

8. Funktionelle Gesundheitslebensmittelzusammensetzung zur Verwendung nach Anspruch 6 oder 7, wobei diese weiter neben dem Wirkstoff mindestens eines ausgewählt aus einem Nahrungsergänzungsmittel, einem Vitamin, einem Elektrolyten, einem Geschmackstoff, einem Farbstoff, einem Verstärker, Pektinsäure und einem Salz davon, Alginsäure und einem Salz davon, einer organischen Säure, einem schützenden kolloidalen Verdickungsmittel, einem Mittel zur Einstellung des pH-Werts, einem Stabilisator, einem Konservierungsstoff, Glycerin, Alkohol und einem Carbonisierungsmittel zur Verwendung in einem kohlensäurehaltigen Getränk umfasst.

## Revendications

1. Composition pharmaceutique destinée à être utilisée dans un procédé de prévention ou de traitement d'un trouble de stress, comprenant un extrait à l'éthanol d'*Hibiscus syriacus* comme ingrédient efficace.

2. Composition pharmaceutique destinée à être utilisée selon la revendication 1, dans laquelle l'extrait à l'éthanol *d'Hibiscus syriacus* est un extrait à l'éthanol de fleur *d'Hibiscus syriacus* ou de racine *d'Hibiscus syriacus.*

3. Composition pharmaceutique pour l'utilisation selon la revendication 1 ou 2, dans laquelle le trouble de stress est une dépression provoquée par le stress.

4. Composition pharmaceutique pour l'utilisation selon l'une quelconque des revendications 1 à 3, dans laquelle elle comprend en outre un véhicule ou un diluant autre que l'ingrédient efficace.

5. Composition pharmaceutique pour l'utilisation selon l'une quelconque des revendications 1 à 4, dans laquelle la composition est administrée par voie orale ou parentérale.

6. Composition alimentaire santé fonctionnelle destinée à être utilisée dans un procédé de prévention ou d'amélioration du stress ou de la dépression provoquée par le stress, comprenant un extrait à l'éthanol d'*hibiscus syriacus* comme ingrédient efficace.

7. Composition d'aliment naturel fonctionnelle destinée à être utilisée selon la revendication 6, dans laquelle l'extrait à l'éthanol *d'Hibiscus syriacus* est un extrait à l'éthanol de fleur *d'Hibiscus syriacus* ou de racine *d'Hibiscus syriacus.*

8. Composition d'aliment naturel fonctionnelle destinée à être utilisée selon la revendication 6 ou 7, dans laquelle elle comprend en outre, autre que l'ingrédient efficace, un supplément nutritionnel et/ou une vitamine et/ou un électrolyte et/ou un arôme et/ou un agent colorant et/ou un agent améliorant et/ou de l'acide pectinique et un sel de celui-ci et/ou de l'acide alginique et un sel de celui-ci et/ou de l'acide organique et/ou un agent épaississant colloïdal protecteur et/ou un agent d'ajustement de pH et/ou un stabilisant et/ou un conservateur et/ou de la glycérine et/ou de l'alcool et/ou un agent carbonatant utilisé pour une boisson gazeuse.
